# EUROPEAN PATENT APPLICATION

(11) **EP 1 290 994 A2**
(43) Date of publication of application: **12.03.2003**
(21) Application number: 02425551.5
(22) Date of filing: 06.09.2002
(51) Int. Cl.: A61F 5/44

(54) **A disposable urinal**

(30) Priority: 10.09.2001 MT 163601
(71) Applicant: Yamamoto Limited, Valletta VLT05 (MT)
(72) Inventor: Buttigieg, Carmel, Santa Venera (MT)
(74) Representative: Bardini, Marco Luigi

(57) **Abstract**

A disposable urinal comprises a urine-storage container (5), a conveyer (6, 14) fit to adhere to the male or female organs for conveying urine to the container (5), and urine-absorbing and gelatinizing material (4) placed within the container (5). The urine absorbing material (4) is housed within a sealed capsule formed within the container (5), and said conveyer (6, 14) comprises means (21, 15) for breaking the capsule as a result of an engagement with the container (5) immediately before use, whereby the material (4) is discharged into the container (5) for reacting with the urine.

## Description

The present invention relates to the field of sanitary appliances. In particular, it concerns a new type of disposable urinal.

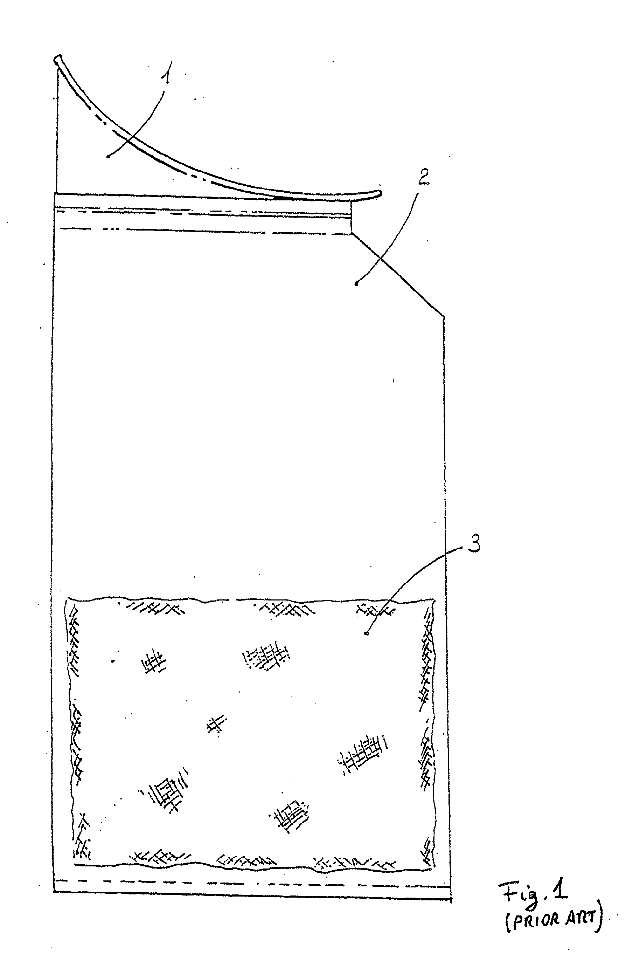

When someone can not move his body normally, micturition becomes a problem, which is normally tackled by the so-called "urinals". At present, a number of solutions exist on the market, generally focused at making the traditional urinal a disposable product. All these solutions are basically formed by three parts, as shown in figure 1: a unisex plastic conveyer 1, a storage plastic bag 2, and a pouch 3 filled with a urine-absorbing and gelatinizing polymer.

These products achieve an unsatisfactory result, for the following reasons.

Firstly, the solderings made in the plastic bag, and between the bag and the conveyer, being made manually, sometimes are not perfect causing a possible dripping.

Besides, it is difficult to direct the bag down when the user is bedridden.

Furthermore, the pouch with the absorbing polymer is very sensitive to the humidity, losing its properties very soon especially if stored where the moisture in the air is high.

Additionally, the reaction time of the polymer is prolonged by the barrier effect of the material used for the pouch, generally paper or non-woven fabric which, under the influence of the liquid pressure is broken into fragments so that the polymer is dispensed. Obviously, in such particular circumstances, a lengthy waiting for the polymer to react can be very annoying.

Moreover, in case of a recycling, the present disposable urinals cause problems, because they are made of two different components, and the expulsion of the urine, which has become a gel as a result of the reaction with the polymer, is difficult because is amalgamated with the fragments of the pouch.

Finally, an aspect to be considered is the production cost. The present disposable urinals are constructed manually according to the following steps:
- the plastic bag is soldered at its top so that a slanting edge is formed;
- the conveyer is soldered to the plastic bag;
- the pouch with the polymer (produced in a different line) is inserted into the bag; and
- the whole product is folded and inserted into another plastic bag, which has to be soldered too.

These operations, which are totally manual, are lengthy (over one minute is required for a single piece), and result in production costs which are too high for a disposable product.

The object of the present invention is to provide a disposable urinal having the following features:
- easy to handle;
- easy to point towards the desired direction;
- easy to recycle;
- low volume when stored;
- no pouch filled with absorbing polymer;
- low reaction time of the absorbing polymer;
- low production cost.

This object is achieved with the disposable urinal according to the present invention, of which the essential characteristics are defined in the main claim attached.

The characteristics and advantages of the disposable urinal in accordance with the present invention will emerge more clearly from the following description of a particular embodiment thereof, which is to be considered as an example and not limitative in any way, said description making reference to the attached drawings of which:
- figure 1 shows a side view of a urinal according to the prior art;
- figures 2a and 2b are side views of the urinal according to the present invention, respectively in the extended and retracted configuration, without the conveyer;
- figures 3 and 4 are axial cross-section views of conveyers of the urinal according to the invention, suitable for male and female users respectively;
- figures 5 to 7 elucidate the operations to be made for connecting the urinal tube to a male conveyer;
- figures 8 to 10 show once again the operations to be made by the user for a proper use of the urinal, the broken and exploded perspective views of figures 8 and 9 showing in greater detail a special polymer-enclosing cap engaged within the neck of the tube according to a different embodiment of the invention;
- figure 11 is a perspective view of a tray for forming a plurality of polymer-enclosing caps in a production process of the urinal according to the embodiment of figures 8 to 10;
- figures 12a to 16 elucidate the production steps of the process making use of the tray of figure 11;
- figures 17 and 18 show the use of the urinal according to the present invention in an underwear equipment for incontinent people; and
- figures 19 and 20 are respective examples of a suspensory bandage of the equipment of figures 17 and 18.

With reference to figures 2a to 7, a tube 5, whose function is the urine storage, is formed by a plurality of convolutions 8 each having an upper ring 8a bigger than the lower ring 8b in order to enter easily each other upon an axial compression of the whole tube.

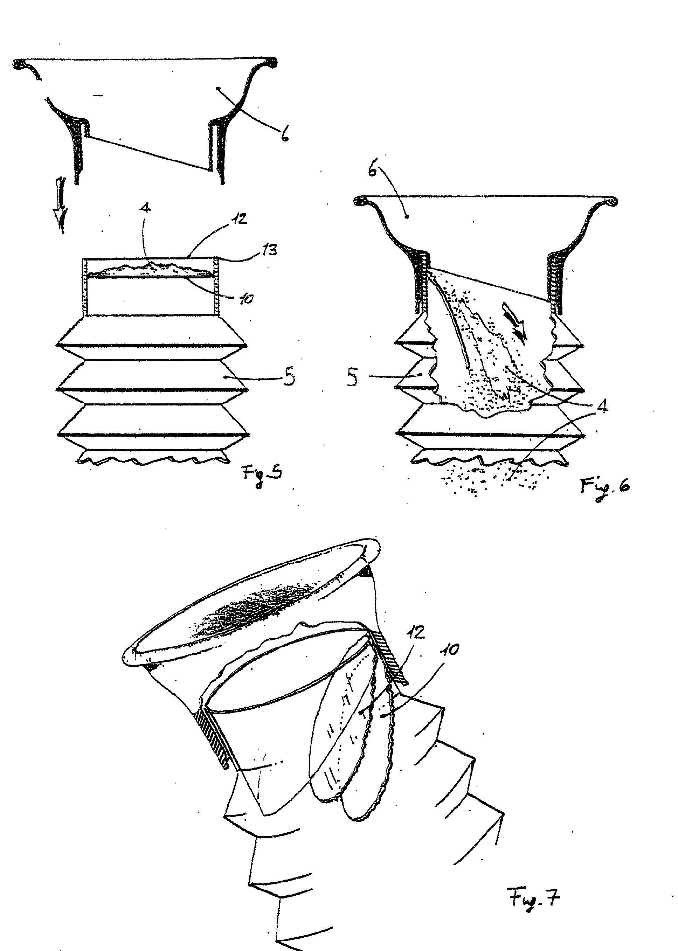

Tube 5 has at its bottom a closed convolution 9 with a bigger diameter. At the opposite end of tube 5 a hollow neck 7 is provided, defining an inlet for the urine. Neck 7 is closed by a membrane 10 and a plastic film 12, the latter soldered to the outer edge 13. Thus, membrane 10 and film 12 define a sealed capsule in which a urine absorbing and gelatinizing polymer 4 is stored (see figure 5), kept dry and thus preserved in the most effective condition.

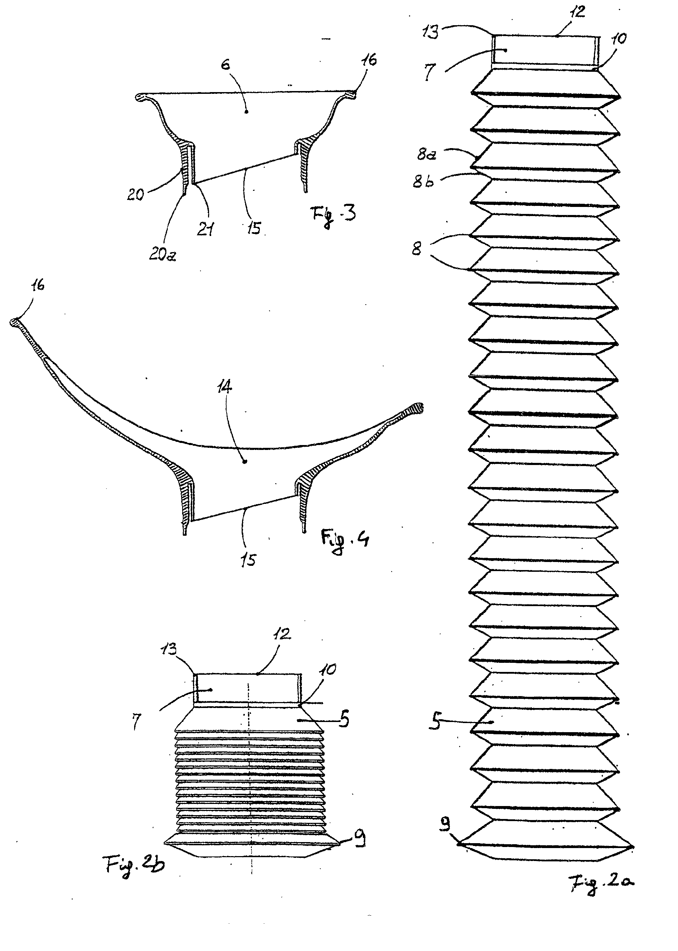

Tube 5 may be produced with a process of blow molding, using a mould having a shape corresponding to the configuration of maximum extension of figure 2a, long enough to store a complete micturition (capacity around 500 milliliters). A male and female organ conveyers 6 and 14 (figures 3 and 4), produced with a process of injection molding, have a top cup-shaped end made with an enlarged edge 16 (larger for the female conveyer), and a bottom end with an inclined spout 15. The bottom end of the conveyer is formed by a couple of coaxial tubes 20, 21 between which edge 13 of neck 7 of tube 5 is fit to be engaged.

Before use, the male/female conveyer, produced with a hard plastic material as polyethylene or polypropylene, is kept separate from tube 5. In use, it is coupled to neck 7 of tube 5 as shown in figures 5 to 7. The user - i.e. a bedridden patient - holds the conveyer in a hand and tube 5 - in a retracted condition - in the other hand. This insertion is easy because of the shape of tubes 20, 21 of the conveyer, being facilitated because outer tube 20 gets thinner towards the free edge 20a, as shown in figures 3 and 4.

Spout 15 progressively enters neck 7, cutting film 12 and membrane 10, allowing polymer 4 to fall down tube 5, which in the meantime has been extended and easily pointed towards the direction which is most favorable for a convenient micturition. Film 12 and membrane 10 are only partially cut, due to the shape of spout 15, and therefore remain in a hinge-like position (see figure 7), obtaining the result that the male/female organ does not touch polymer 4 (because of the protection of film 12), and the urine does not drip out (because of the barrier effect of membrane 10) during micturition.

In this case polymer 4 will work in the best condition, being free inside tube 5 and not trapped or fixed into layers of papers or non-woven fabric as in all the other applications on the market. Therefore, its reaction time is very short. After micturition has been fulfilled, the disposal and possible recycling of the urinal is trouble-free, since the whole device is basically made of a single type of material (plastic) and no paper fragments are amalgamated with the gelatinized urine.

Polymer 4, when put inside, falls down at the bottom end of tube 5. In this case the property of an immediate gelatinizing is reduced because the urine gelatinizes the exposed surface but not the core of the material. For this reason, when the process of blow-molding is carried out, also an oily substance (e.g. an essential oil) will be blown inside tube 5. With this operation the polymer will adhere around the whole internal surface of tube 5 and progressively will gelatinize the urine.

Once tube 5 is produced falls into a assembly line where the following steps are carried out:
- compression of tube 5;
- insertion of polymer 4 into neck 7;
- soldering of film 12 on edge 13 of neck 7; and
- falling into the final packaging station.

The production of this kind of urinal is simple and does not provide any manual operation because made in a proper assembly line, and therefore the relevant production costs are very low. Furthermore, the absence of solderings in tube 5 avoids any possibility of urine dripping.

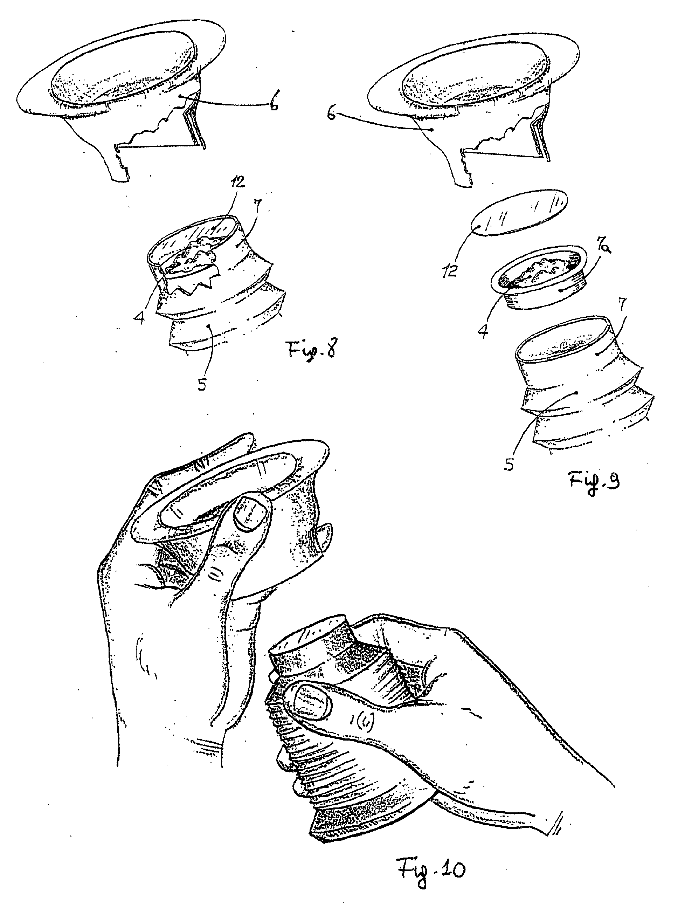

As shown in figures 8 to 14, instead of filling neck 7 with the polymer, a cap 7a can be produced, having a bottom 10a corresponding to membrane 10 of the previous embodiment, and filled with polymer 4. Cap 7a, once filled with the polymer will be covered by the same film 12, soldered on its edge 13a. Obviously, cap 7a will be inserted into neck 7 which, in this case, is in itself totally open.

Figures 11 to 16 show in particular how caps 7a may be produced and assembled to tubes 5. The process starts from a tray 40 obtained from thin sheets or rolls of rigid plastic material. Tray 40 has a plurality of cavities 41 each corresponding to a cap 7a. Cavities 41 may be formed by suction after heating, and consequently softening the plastic material. Afterwards, the material is cooled by compressed air or by ventilators. With this vacuum forming process very thin caps can be obtained: caps are very light and bottom 10a is easily breakable. Besides, the productivity is remarkably high, because a tray incorporates a number of caps.

Subsequently, in an assembly line, cavities 41 are filled with polymer 4 (figure 12b), and a plastic film 12 is soldered over the whole tray (figure 12c). Then, the tray passes through a press which perforates the material along circular paths 42 (figure 11) enclosing each and every cavity 41 formed in the tray.

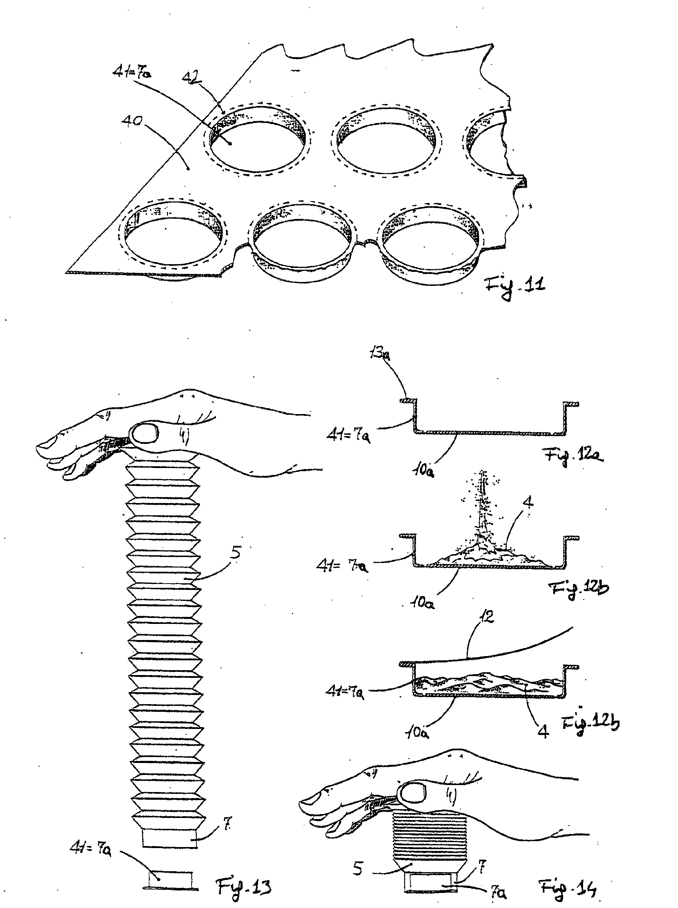

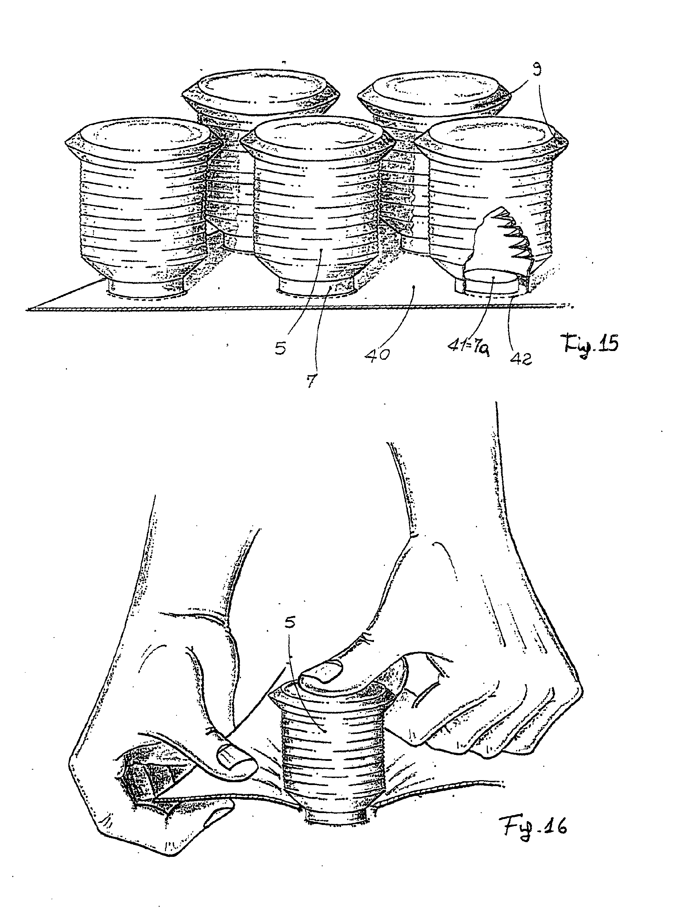

Necks 7 of tubes 5 in the extended condition are engaged around the outer, convex side of respective cavities 41 (figure 13), tubes 5 being compressed (figure 14).

Eventually, the tray is full of single urinal tubes 5 compressed on the outside of cavities 41 - i.e. caps 7a - and ready for use (see figure 15). Each tube 5 is easily wrenched away from the tray, thanks to perforation 42 (figure 16).

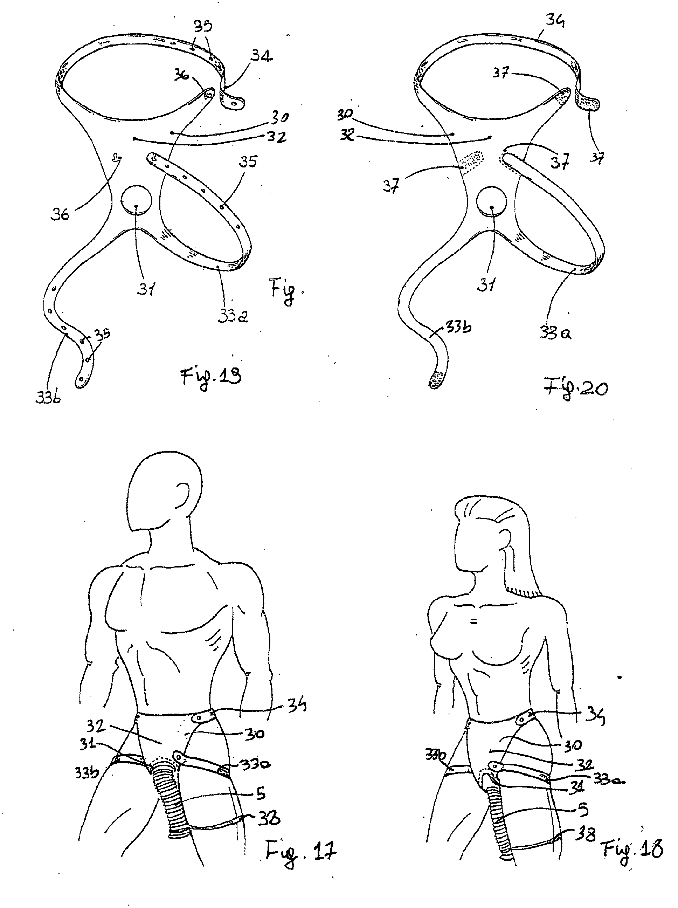

Referring now to figures 17 to 20, the urinal according to the invention can also be used by incontinent people. In this case the top part of the conveyer 6, 14 is preferably made of a soft material as latex or polyurethane or thermoplastic rubber or similar, because incontinent people may stand on during the day and can have the urinal adhering to the genital area for some hours. Moreover, tube 5 can be narrower and longer than for the previous application, in order to result more comfortable for a standing user.

The retractable tube 5 is associated to a suspensory bandage 30 (see figures 19 and 20) with a front part 32 in contact with the belly. A hole 31 for engagement with tube 5 is formed in front part 32 in correspondence to the genital area. Three belts extend from front part 32: lower ones 33a and 33b wind round the respective legs of the user, whereas an upper belt 34 winds around the waist.

Once tube 5 is inserted into hole 31 and directed down along the legs, is kept in position because the enlarged edge 16 of male or female conveyer 6, 14 can not pass through the hole. The conveyer adheres to the male/female organ thanks to the suspensory belts whose tension can be varied via an adjustable fastening system. Such fastening may be carried out by pins 36 sewed or glued to front part 32, and engageable with holes 35 formed in belts 33a, 33b and 34. Alternatively, Velcro™ or similar detachable elements 37 may be used.

The suspensory bandage can be replaced by normal underpants on which a hole is formed which allows the passage of tube 5 but not of conveyer 6, 14. However, in this case the conveyer of male-female organ may not have a perfect adhesion as with the suspensory. For a more convenient use, tube 5 may be fixed to a leg of the user via a string 38.

In light of the above, the urinal according to the invention can substitute the external catheters and the relevant urine-collecting bags. The retractable tube 5, being formed by a plurality of convolutions, can make a bellows-effect. If the user compresses and releases the tube, as a pump, when the urinal is in the right position adhering perfectly to the genital area, the tube can suck the urine in those patients suffering of lack of micturition, avoiding in some cases the use of bladder catheters.

In all the embodiments, the nature of the absorbing polymer may vary and consequently also the relevant reaction time. Products already used for this kind of purposes, as in sanitary napkins, can be exploited. Examples of polymers that can be used are:
- cellulose-acrylonitrile;
- carboxymethylated polysaccharide acrylonitrile and in general all the by-products of the polyacrilic acid and of the polyacrilonitrile.

Also natural components can be used for gelatinizing the urine, such as dried and pulverized algae even if, in this case, the reaction time is prolonged.

Variations and/or modifications can be brought to the disposable urinal in accordance with the present invention without going beyond the scope of the invention as defined by the claims hereinbelow.

## Claims

1. A disposable urinal comprising a urine-storage container (5), a conveyer (6, 14) fit to adhere to the male or female organ for conveying urine to said container (5), and urine-absorbing and gelatinizing material (4) placed within said container (5), **characterized in that** said urine absorbing material (4) is housed within a sealed capsule formed within said container (5), and **in that** said conveyer (6, 14) comprises means (21, 15) for breaking said capsule as a result of an engagement with said container (5) immediately before use, whereby said material is discharged into said container (5) for reacting with the urine.

2. The urinal according to claim 1, wherein said container (5) is a retractable tube formed by a plurality of convolutions (8), said tube assuming a retracted position before use and for engagement with said conveyer (6, 14), and an extended position in use so that it may be pointed towards any desired directions, said capsule being located in correspondence to an inlet hollow neck (7) of said tube (5) fit to engage with said conveyer (6, 14).

3. The urinal according to claim 2, wherein said capsule is defined between a membrane (10) and a plastic film (12) sealingly closing said inlet hollow neck (7).

4. The urinal according to claim 2, wherein said capsule is defined by a cap (7a) comprising a bottom (10a), said cap (7a) being sealingly closed by a plastic film (12) and engaged within said neck (7).

5. The urinal according to any of claims 3 and 4, wherein said conveyer (6, 14) comprises a top cup-shaped end with an enlarged edge (16) and a bottom end with a spout (15) for breaking said capsule, said bottom end being formed by a couple of coaxial tubes (20, 21) between which said neck (7) of said tube (5) is fit to be engaged.

6. The urinal according to claim 5, wherein said spout (15) is inclined so that when progressively entering said neck (7), said film (12) and said membrane (10) or said bottom (10a) are only partially broken and remain in a hinge-like position, whereby the male/female organ does not touch said material and the urine does not drip out.

7. The urinal according to any of the previous claims, wherein said container (5) also contains an oily substance, e.g. an essential oil, distributed over the internal surface thereof, in order to assist the reaction between said absorbing material and the urine.

8. The urinal according to any of the previous claims, wherein said urine absorbing material is a polymeric material chosen among the following: cellulose-acrylonitrile; carboxymethylated polysaccharide acrylonitrile; and in general all the by-products of the polyacrilic acid and of the polyacrilonitrile.

9. The urinal according to any of claims 1 to 7, wherein said urine absorbing material is a dried and pulverized algae.

10. The urinal according to any of claims 2 to 9, comprising also an underwear garment (30) to be worn by an incontinent user, said garment comprising a front part (32) with a hole (31) allowing the passage of said tube (5) but not of said conveyer (6, 14), so that said tube (5) is assured in correspondence of the genital area of the user.

11. The urinal according to claim 10, wherein said garment (30) is a suspensory bandage comprising at least three belts extending from said front part (32), two belts (33a, 33b) winding around respective legs of the user and one (34) winding around the waist, said belts being adjustably assured to said front part (32) by a pins (36) and holes (35) engagement system or Velcro™ elements (37).

12. The urinal according to claim 10 or 11, wherein said tube (5) is fixed to a leg by a string.

13. A process for forming tubes (5) of urinals according to any of the claims from 3 to 9, comprising the following steps:
- said tubes (5) are produced with a process of blow molding, using a mould having a shape corresponding to the configuration of maximum extension;
- said tubes are compressed;
- said absorbing material (4) is inserted into said neck (7) ;
- said plastic film (12) is soldered on said neck (7); and
- said tubes (5) fall into a final packaging station.

14. A process for forming tubes (5) of urinals according to any of the claims from 4 to 9, comprising the following steps:
- a plurality of cavities (41) are formed in a tray (40) of rigid, thin sheet-like plastic material, each cavity (41) corresponding to said cap (7a);
- said cavities (41) are filled with said absorbing material (4);
- said plastic film (12) is soldered over the whole tray (40) ;
- circular perforations (42) enclosing respective cavities (41) are formed in said tray (40) ;
- said tubes (5) are produced with a process of blow molding, using a mould having a shape corresponding to the configuration of maximum extension;
- said tubes (5) in the extended condition are engaged with the relevant necks (7) around the outer, convex side of respective cavities (41), being compressed and assuming a retracted condition (figure 14);
- each of said tubes (5) is wrenched away from said tray (40) along the relevant perforated path (42);
- said tubes fall into a final packaging station.
